## Europäisches Patentamt
## European Patent Office
## Office européen des brevets

(11) Numéro de publication: **0 102 853**
**B1**

(12) **FASCICULE DE BREVET EUROPEEN**

(45) Date de publication du fascicule du brevet: **13.07.88**

(51) Int. Cl.⁴: **A 61 F 2/60**

(21) Numéro de dépôt: **83400159.6**

(22) Date de dépôt: **24.01.83**

(54) **Dispositif de recherche d'alignement, d'alignement et d'orientation pour les prothèses des membres inférieurs.**

(30) Priorité: **30.07.82 FR 8213332**

(43) Date de publication de la demande:
**14.03.84 Bulletin 84/11**

(45) Mention de la délivrance du brevet:
**13.07.88 Bulletin 88/28**

(84) Etats contractants désignés:
**AT BE CH DE GB IT LI LU NL SE**

(56) Documents cités:
**DE-A-1 566 404**
**FR-A-1 445 102**
**US-A-3 422 462**
**US-A-3 538 516**

(73) Titulaire: **ETABLISSEMENTS PROTEOR**
**11 rue des Buttes**
**F-21100 Dijon (FR)**

(72) Inventeur: **Palfray, Michel-Raymond**
**Route de Pagny**
**F-21250 Seurre (FR)**

(74) Mandataire: **Madeuf, René Louis et al**
**Cabinet Madeuf 3, Avenue Bugeaud**
**F-75116 Paris (FR)**

EP 0 102 853 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Description

La présente invention concerne un nouveau dispositif de recherche d'alignement, d'alignement et d'orientation pour des prothèses des membres inférieurs.

La technique antérieure a fait connaître le DE—1 566 404 qui comporte une couronne conique déplaçable dans une gorge en V d'un noyau au moyen de vis pour régler la position du noyau par rapport à un plateau et par conséquent cette réalisation permet un réglage d'alignement.

Le US—A—3 538 516 décrit un empilage de pièces prenant appui les unes sur les autres alternativement par des surfaces planes et courbes, lesdites pièces étant déplaçables par des vis. Par ce moyen, un réglage d'alignement et d'angle est possible par approches successives.

Le US—A—3 422 462 décrit un bloc métallique creux dont deux faces opposées présentent des fentes percées à angle droit pour le passage de vis respectivement vissées dans deux éléments de prothèse, l'élément supérieur étant conformé de façon sphérique pour rendre possible un réglage de l'angle fait par la partie inférieure de la prothèse par rapport à la partie supérieure.

Tous les dispositifs connus sont métalliques, donc lourds.

Le dispositif de l'invention est destiné à permettre des réglages d'alignement et/ou d'orientation d'une partie de prothèse par rapport aux autres, par exemple, de l'emboîtement d'une prothèse pour amputation de cuisse ou de jambe afin d'obtenir les meilleures positions statiques et dynamiques pour la marche des patients.

La réalisation du dispositif de l'invention permet, en outre, une fabrication bon marché extrêmement légère. De plus, le dispositif de l'invention, lorsqu'il a été réglé, peut être complètement noyé dans une forme simulant l'aspect extérieur du membre amputé.

Conformément à l'invention, le dispositif d'alignement de deux parties consécutives d'une prothèse de membre inférieur d'un amputé, ledit dispositif faisant partie intégrante de la prothèse et comprenant au moins trois éléments empilés les uns sur les autres dont les deux extrêmes sont reliés respectivement aux parties de prothèse inférieure et supérieure et présentent chacun une face de contact avec au moins un élément intermédiaire contigu, ladite face de contact étant de forme plane ou courbe, des moyens étant prévus pour immobiliser entre eux les éléments du dispositif après leurs déplacements relatifs, est caractérisé en ce qu'une face plane de contact est formée sur l'élément relié à la partie inférieure de la prothèse, en ce qu'une première face courbe de contact est formée sur l'élément fixé à la partie supérieure de prothèse et en ce qu'au moins une deuxième face courbe de contact avec ladite première face courbe de contact est formée sur un élément intermédiaire, le rayon de courbure de chaque face courbe de contact présentant une longueur correspondant approximativement à la distance séparant ladite face courbe de contact du centre articulaire susjacent de la partie supérieure de la prothèse de l'amputé.

L'invention concerne aussi un procédé pour l'alignement, l'une par rapport à l'autre, de deux parties d'une prothèse de membre inférieur d'un amputé, caractérisé en ce qu'on dispose entre lesdites deux parties des moyens de réglage d'alignement et en ce qu'on immobilise lesdits moyens de réglage d'alignement après réglage pour qu'ils fassent partie intégrante de la prothèse, en ce qu'on dispose, en outre, entre lesdites deux parties des moyens de réglage de l'orientation d'une des parties par rapport à l'autre et en ce qu'on immobilise lesdits moyens de réglage d'orientation après réglage pour qu'ils fassent partie intégrante de la prothèse et, en ce que supplémentairement on rectifie les génératrices externes des moyens de réglage après leur fixation définitive et en ce qu'on les enveloppe pour qu'ils fassent partie intégrante de la prothèse.

Diverses autres caractéristiques de l'invention ressortent d'ailleurs de la description détaillée qui suit.

Des formes de réalisation de l'objet de l'invention sont représentées, à titre d'exemples non limitatifs, au dessin annexé.

La fig. 1 est une élévation schématique de face d'un amputé de la cuisse.

La fig. 2 est une élévation analogue à la fig. 1 illustrant le dispositif d'alignement de l'invention de façon schématique.

La fig. 3 est une perspective éclatée du dispositif de l'invention selon un premier mode de réalisation.

La fig. 4 est une perspective éclatée du dispositif de l'invention selon un second mode de réalisation.

La fig. 5 est une élévation de face, à plus grande échelle, montrant le dispositif et selon un mode de réalisation préféré.

La fig 6 est une coupe suivant la ligne VI-VI de la fig. 5.

La fig. 7 est une élévation montrant le dispositif dans son application à une prothèse de cuisse.

La fig. 8 est une élévation schématique montrant l'application du dispositif à une prothèse de jambe.

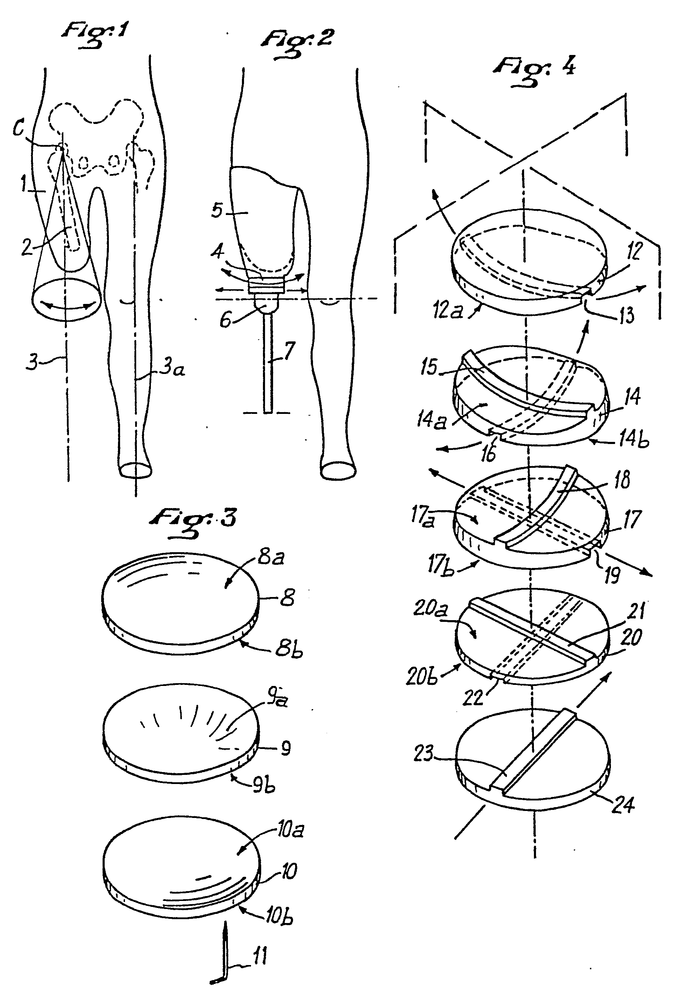

La fig. 1 montre de face un amputé de la cuisse droite, 1 désignant le moignon et 2 le segment de fémur, tant le moignon que le fémur présentant une déviation par rapport à la ligne verticale figurée en 3 en ce qui concerne le moignon et en 3a en ce qui concerne la jambe saine.

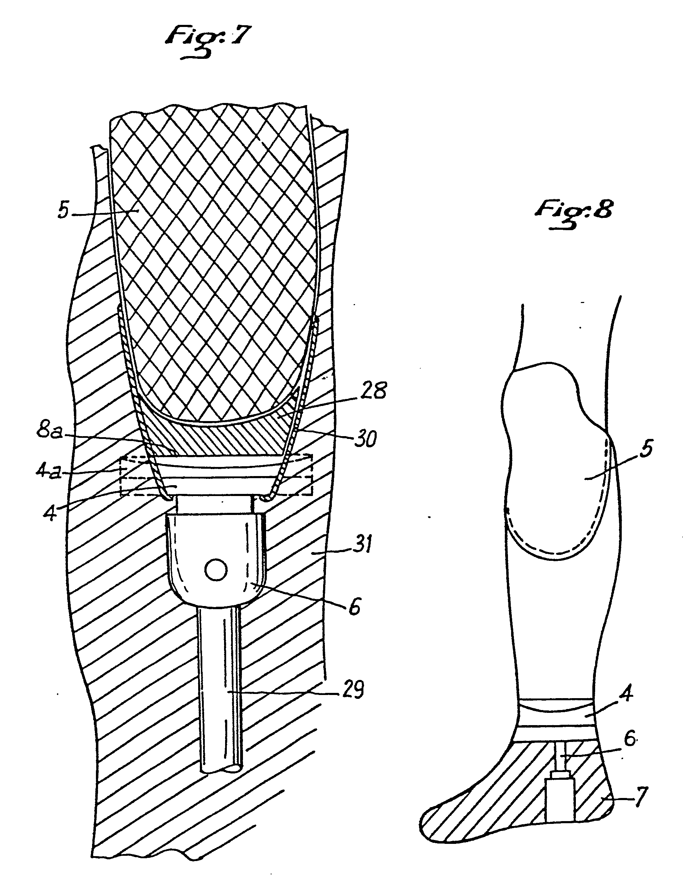

Pour permettre un appareillage correct de l'amputé, l'invention crée un dispositif de recherche d'alignement, d'alignement et d'orientation désigné dans son ensemble par 4. Ce dispositif est fixé à la partie inférieure de l'emboîture 5 qui peut être de cuisse comme montré par les fig. 2 et 7 ou de jambe comme montré par la fig. 8 ou à toute autre partie de la prothèse. Le dispositif de l'invention est, par ailleurs, relié à un mécanisme 6 faisant partie de la prothèse 7, ce mécanisme pouvant être une articulation de genou ou de pied comme l'illustrent les fig. 2, 7 et 8 ou à une autre partie de la prothèse.

La fig. 3 illustre une première réalisation du dispositif qui comporte trois éléments 8, 9 et 10. L'élément 8 présente un dessus 8a destiné à être relié à une partie de prothèse et un dessous 8b convexe. Le disque 9 présente un dessus 9a concave de même rayon de courbure que le dessous convexe 8b de l'élément 8. Le dessous 9b de l'élément 9 est plan de même que le dessus 10a de l'élément 10 dont le dessous 10b est destiné à être relié par exemple au mécanisme 6 ou à toute autre partie de prothèse.

Le centre du rayon de courbure de la surface convexe de l'élément 8 et de la surface concave de l'élément 9 est proche du centre articulaire de l'articulation sus-jacente C de la prothèse de l'amputé.

En déplaçant l'élément 9 par rapport à l'élément 8, il est possible d'effectuer un réglage de l'angle du moignon par rapport à la ligne verticale 3 tant dans le plan frontal que dans le plan sagittal ainsi que dans tous les plans intermédiaires. Par ailleurs, le déplacement de la face plane 10a de l'élément 10 contre la face plane 9b de l'élément 9 permet de déplacer la ligne de charge tant par rapport au plan frontal qu'au plan sagittal pour tenir compte de la morphologie particulière du patient à appareiller et donc d'assurer l'alignement de la prothèse.

Les éléments décrits ci-dessus en référence à la fig. 3 sont avantageusement fabriqués en une matière aussi légère que possible et, de préférence, en matière plastique chargée de matière aimantée, par exemple un caoutchouc imprégné de ferrite aimantée. Cette disposition permet d'effectuer les réglages nécessaires puis les éléments sont bloqués entre eux par exemple au moyen d'une goupille telle que celle illustrée en 11 à la fig. 3. Une rectification du pourtour des éléments déplacés les uns par rapport aux autres peut aussi être effectuée pour que leurs génératrices externes soient alignées avec celles de l'emboîture 5 et de la prothèse. L'ensemble du dispositif peut ensuite être noyé dans de la résine ou entouré, ainsi au moins que le bas de l'emboîture et le haut de la prothèse, par des bandes adhésives pour assurer une immobilisation définitive.

La fig. 4 illustre une variante de réalisation permettant une fabrication du dispositif en toute matière désirée par exemple en matière plastique. Dans cette variante, le dispositif comporte: un premier élément 12 dont la face inférieure 12a est convexe et dans laquelle est formée une rainure 13, un second élément 14 dont le dessus 14a est concave et présente une nervure 15 qui coulisse dans la rainure 13, le dessous 14b de l'élément 14 étant lui-même convexe et présentant une rainure 16 qui est orthogonale par rapport à la nervure 15 engagée dans la rainure 13 de l'élément 12. Un troisième élément 17 présente, comme l'élément 14, un dessus 17a concave et une nervure 18 correspondant à la rainure 16 de l'élément 14. Le dessous de l'élément 17 est plan et présente une rainure 19 orthogonale à la nervure 18. Un quatrième élément 20 dont les dessus et dessous 20a, 20b sont plans présente sur son dessus une nervure 21 qui correspond à la rainure 19 de l'élément 17 et sur son dessous une rainure 22 pour coopérer avec une nervure 23 formée dans un cinquième élément 24 dont le dessus est plan.

Comme cela ressort de ce qui précède, le déplacement de l'élément 24 par rapport à l'élément 20 peut s'effectuer dans un plan tandis que le déplacement de l'élément 20 par rapport à l'élément 17 peut s'effectuer dans un plan perpendiculaire. Les nervures et rainures correspondantes de ces éléments étant orthogonales, tous les réglages d'aplomb peuvent donc être effectués en fonction de la morphologie de l'amputé à appareiller.

Le déplacement relatif qui peut être conféré à l'élément 17 par rapport à l'élément 14 et à celui-ci par rapport à l'élément 12 permet, par ailleurs, du fait de la position relative de leurs nervures et rainures d'effectuer tous les réglages d'orientation nécessaires.

Le mode de réalisation décrit permet une fabrication par moulage ou usinage ou tout autre mode permis par la technique de pièces en bois ou matière plastique ou en tout autre matière et la fixation des pièces entre elles peut être assurée par collage, goupillage ou tout autre moyen de la technique.

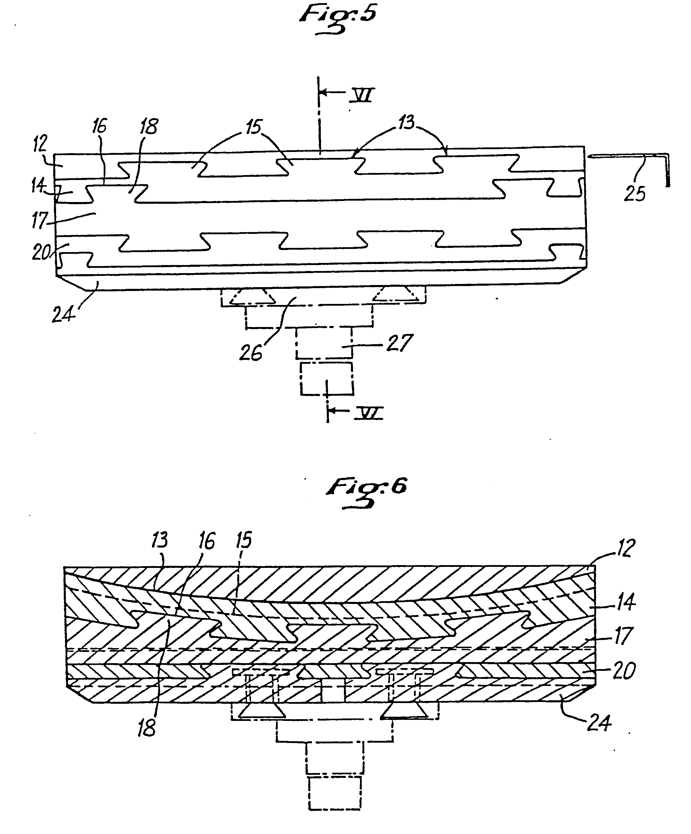

Les fig. 5 et 6 illustrent un développement du mode de réalisation de la fig. 4, développement suivant lequel les éléments 12, 14, 17, 20 et 24 présentent chacun des rainures 13, des nervures 15, des rainures 16, des nervures 18, etc..., qui sont en forme de queue d'aronde et qui sont alternées, de sorte que les éléments successifs sont guidés dans deux plans perpendiculaires par l'ensemble des assemblages en queue d'aronde.

Au cours des réglages intermédiaires auxquels procède le prothésiste, les différents éléments peuvent être immobilisés les uns par rapport aux autres au moyen de broches 25, de vis ou de tout autre moyen approprié, ceci jusqu'à l'obtention du réglage final.

Dans un mode d'application à la réalisation d'une prothèse de cuisse, on fixe provisoirement, par exemple par collage, le dessus du premier élément 8 ou 12 à la base 28 de l'emboîture 5, puis le dernier élément 24 sur le raccord 26 d'un élément 6, par exemple par vissage et on effectue les reglages décrits ci-dessus. On procède ensuite à un usinage du bord périphérique du dispositif 4 pour ajuster la périphérie 4a faisant saillie par rapport à la base 28 de l'emboîture.

Ensuite, les éléments sont bloqués et fixés définitivement à l'emboîture 5. Un mode de réalisation consiste à enrouler autour de celle-ci des rubans 30 imprégnés de matière plastique pour réaliser une stratification formant liaison entre l'emboîture et le dispositif. Finalement, un garnissage 31 est mis en place de façon connue en soi pour envelopper l'ensemble de l'armature de la prothèse et le dispositif en silhouettant une cuisse, un genou et une jambe naturelle.

Dans ce qui précède les surfaces concaves ou convexes sont indifféremment à génératices sphériques ou cylindriques.

La fig. 8 montre une application qui est faite du dispositif à une prothèse de jambe; dans ce cas le dispositif est monté immédiatement au-dessus du pied. Comme précédemment après réglage, le pourtour du dispositif est modifié pour s'adapter exactement à la forme extérieure que doit présenter la prothèse.

L'invention n'est pas limitée aux exemples de réalisation représentés et décrits en détail, car diverses modifications peuvent y être apportés sans sortir de son cadre tel qu'illustré par les revendications ci-jointes.

**Revendications**

1. Dispositif d'alignement de deux parties consécutives (5, 6) d'une prothèse de membre inférieur d'un amputé, ledit dispositif faisant partie intégrante de la prothèse et comprenant au moins trois éléments empilés les uns sur les autres (8—9—10, 12—14—17—20—24) dont les deux extrêmes sont reliés respectivement aux parties de prothèse inférieure (6) et supérieure (5) et présentent chacun une face de contact avec au moins un élément intermédiaire contigu (9 ou 14, 17, 20), ladite face de contact étant de forme plane ou courbe, des moyens étant prévus pour immobiliser entre eux les éléments du dispositif après leurs déplacements relatifs, caractérisé en ce qu'une face plane de contact est formée sur l'élément (10, 24) relié à la partie inférieure (6) de la prothèse, en ce qu'une première face courbe de contact est formée sur l'élément (8, 12) fixé à la partie supérieure (5) de prothèse et en ce qu'au moins une deuxième face courbe de contact avec ladite première face courbe de contact (9b—17a, 14b—14a, 12a) est formée sur un élément intermédiaire (9 ou 17—14), le rayon de courbure de chaque face courbe de contact présentant une longueur correspondant approximativement à la distance séparant ladite face courbe de contact du centre articulaire sus-jacent (c) de la partie supérieure (5) de la prothèse de l'amputé.

2. Dispositif suivant la revendication l, caractérisé en ce qu'il comporte trois éléments (8, 9, 10) empilés les uns sur les autres, des moyens étant prévus pour maintenir entre eux lesdits éléments pendant le déplacement de l'élément intermédiaire (9) pour modifier, d'une part, le décalage axial des parties de prothèse (5, 6) et, d'autre part, l'inclinaison relative desdites parties de prothèse.

3. Dispositif suivant l'une des revendications 1 et 2, caractérisé en ce que les éléments (8, 9, 10) sont reliés par des moyens magnétiques empêchant un arrachement accidentel pendant le déplacement de l'élément intermédiaire (9) par rapport aux éléments (8, 10) fixés aux parties de prothèse (5, 6).

4. Dispositif suivant la revendication 1, caractérisé en ce qu'il comporte un premier élément (12) fixé à la partie supérieure de prothèse (5) et présentant une face inférieure convexe (12a), un second élément (14) présentant sur son dessus une face concave (14a) et sur son dessous une face convexe (14b), un troisième élément (17) présentant sur son dessus une face concave (17a) et sur son dessous une face plane, un quatrième élément (20) présentant sur son dessus et sur son dessous des faces planes et un cinquième élément (24) fixé à la partie inférieure (6) de prothèse et présentant sur son dessus une face plane.

5. Dispositif suivant l'une des revendications 1 et 4, caractérisé en ce que les faces concaves et convexes sont des portions de surfaces cylindriques.

6. Dispositif suivant l'une des revendications 1 à 4, caractérisé en ce que les faces concaves et convexes sont des surfaces sphériques.

7. Dispositif suivant l'une des revendications 1 à 6, caractérisé en ce que les éléments sont fabriqués en bois, métal, matière plastique et autre matière et comprennent des moyens de guidage assurant leur liaison mutuelle.

8. Dispositif suivant l'une des revendications 1, 2 et 5, 6, caractérisé en ce que les éléments présentent sur leurs faces en contact des moyens de guidage orthogonaux (13, 15, 16, 18, 19, 21, 22, 23) en queue d'aronde.

9. Dispositif suivant l'une des revendications 1 à 8, caractérisé en ce qu'il est fixé aux éléments de prothèse (5, 6) par un ruban (30) enroulé et imprégné de matière plastique.

10. Procédé pour l'alignement, l'une par rapport à l'autre, de deux parties d'une prothèse de membre inférieur d'un amputé mettant en oeuvre le dispositif de l'une des revendications 1 à 9, caractérisé en ce qu'on dispose entre lesdites deux parties les moyens d'alignement réglables et en ce qu'on immobilise lesdits moyens d'alignement après réglage pour qu'ils fassent partie intégrante de la prothèse, en ce qu'on dispose, en outre, entre lesdites deux parties les moyens de réglage de l'orientation d'une des parties par rapport à l'autre et en ce qu'on immobilise lesdits moyens de réglage d'orientation après réglage pour qu'ils fassent partie intégrante de la prothèse et en ce que, supplémentairement, on rectifie les génératrices externes des moyens de réglage après leur fixation définitive et en ce qu'on les enveloppe pour qu'ils fassent partie intégrante de la prothèse.

**Patentansprüche**

1. Vorrichtung zum Ausfluchten von zwei aufeinanderfolgenden Prothesenteile (5, 6) für eine untere Gliedmasse eines Amputierten, wobei die Vorrichtung einen integrierenden Bestandteil der Prothese bildet und wenigstens drei aufeinander geschichteten Elemente (8—9, 10—12—14—17—20—24) umfasst, von denen die beiden äussersten Elemente jeweils mit dem unteren (6) und dem oberen (5) Prothesenteil verbunden sind und jeweils eine Berührungsfläche mit wenigstens einem angrenzenden Zwischenelement (9 oder 14, 17, 20) aufweist, wobei die Berührungsfläche eben oder gekrümmt ist und Mittel vorgesehen sind, um die Elemente der Vorrichtung nach ihren Relativverschiebungen aneinander zu halten, dadurch gekennzeichnet, dass eine ebene Berührungsfläche auf dem mit dem unteren Teil (6) der Prothese verbundenen Element (10, 24) gebildet ist, dass eine erste gekrümmte Berührungsfläche auf dem mit dem oberen Teil (5) der Prothese verbundenen Element (8, 12) gebildet ist und dass wenigstens eine zweite gekrümmte, die erste Berührungsfläche (9b—17a, 14b—14a, 12a) berührende Berührungsfläche auf einem Zwischenelement (9 oder 17—14) gebildet ist, wobei der Krümmungsradius jeder gekrümmten Berührungsfläche eine Länge hat, die näherungsweise der Entfernung entspricht, welche diese Berührungsfläche vom darüberliegenden Gelenkmittelpunkt (c) des oberen Teils (5) der Prothese des Amputierten trennt.

2. Vorrichtung nach Anspruch 1, dadurch gekennzeichnet, dass sie drei aufeinander geschichtete Elemente (8, 9, l0) umfasst, wobei Mittel vorgesehen sind, um die Elemente während der Verschiebung des Zwischenelementes (9) aneinander zu halten, um so einerseits die axiale Verschiebung der Prothesenteile (5, 6) und andererseits die relative Neigung der Prothesenteile zu beeinflussen.

3. Vorrichtung nach einem der Ansprüche 1 oder 2, dadurch gekennzeichnet, dass die Elemente (8, 9, l0) durch magnetische Mittel verbunden sind, die während der Verschiebung des Zwischenelements (9) gegenüber den mit den Prothesenteilen (5, 6) befestigten Elementen (8, 10) jedes zufällige Lossreissen verhindern.

4. Vorrichtung nach Anspruch 1, dadurch gekennzeichnet, dass sie ein erstes Element (12) umfasst, welches an den oberen Prothesenteil (5) befestigt ist und eine konvexe Unterseite (12a) aufweist, ein zweites Element (14) welches oben eine konkave Seite (14a) und unten eine konvexe Seite (14b) besitzt, ein drittes Element (17) mit einer oben gelegenen konkaven Seite (17a) und einer unten gelegenen ebenen Seite, ein viertes Element (20) mit oben und unten parallelen Seiten sowie ein fünftes Element (24), welches an einem anderen Prothesenteil (6) befestigt ist und oben eine ebene Seite aufweist.

5. Vorrichtung nach einem der Ansprüche 1 oder 4, dadurch gekennzeichnet, dass die konkaven und konvexen Seiten Zylinderflächenabschnitte sind.

6. Vorrichtung nach einem der Ansprüche 1 bis 4, dadurch gekennzeichnet, dass die konkaven und konvexen Seiten sphärische Flächen sind.

7. Vorrichtung nach einem der Ansprüche 1 bis 6, dadurch gekennzeichnet, dass die Elemente aus Holz, Kunststoff oder einem anderen Material gefertigt sind und Führungsmittel aufweisen, die ihre gegenseitige Verbindung gewährleisten.

8. Vorrichtung nach einem der Ansprüche 1, 2 oder 5, 6, dadurch gekennzeichnet, dass die sie bildenden Elemente an ihren Berührungsflächen orthogonale, schwalbenschwanzförmige Führungsmittel (13, 15, 16, 18, 19, 21, 22, 23) aufweisen.

9. Vorrichtung nach einem der Ansprüche 1 bis 8, dadurch gekennzeichnet, dass sie an die Prothesenteile (5, 6) über ein aufgewickeltes, mit Kunststoff imprägniertes Band (30) befestigt ist.

10. Verfahren zur gegenseitigen Ausrichtung der beiden Teile einer Prothese für eine untere Gliedmasse eines Amputierten, bei welchem die in einem der Ansprüche 1 bis 9 beschriebene Vorrichtung benutzt wird, dadurch gekennzeichnet, dass man zwischen der beiden Teilen Mittel zum Einstellen der Ausrichtung anordnet und diese Mittel zur Ausrichtung nach der Einstellung fixiert, so dass sie einen integrierenden Bestandteil der prothese bilden, dass man zusätzlich zwischen den beiden Prothesenteilen Mittel zur Einstellung der Orientierung eines der Teile relativ zum andern anordnet und diese Mittel zum Einstellen der Ausrichtung nach der Einstellung fixiert, so dass sie einen integrierenden Bestandteil der Prothese bilden und dass man zusätzlich die äusseren Erzeugenden der Einstellmittel nach ihrer endgültigen Fixierung begradigt und sie umhüllt, so dass sie einen integrierenden Bestandteil eines Prothesenteils bilden.

**Claims**

1. Device for the alignment of two consecutive portions (5, 6) of a prosthesis of an amputated person lower limbs, said device being an integral part of the prosthesis and comprising at least three elements stacked upon each other (8—9—10, 12—14—17—20—24), the end elements of which are respectively connected to the lower (6) and upper (5) prosthesis portions and each have a contact face with at least one adjacent intermediary element (9 or 14, 17, 20), said contact face having a plane or curved shape, means being provided for maintaining together the elements of the device after their relative displacement characterized in that a plane contact face is formed on the element (10, 24) connected to the lower portion (6) of the prosthesis, in that a first curved contact face is formed on the element (8, 12) fixed at the upper portion (5) of the prosthesis and in that at least a second curved contact face contacting said first curved contact face (9b, 17a, 14b, 14a, 12a) is formed on an intermediary element (9 or 17—14), the radius of curvature of each curved contact face having a length corresponding substantially to the distance separating said curved contact face from the overlying articular center (c) of the upper portion (5) of the prosthesis of the amputated person.

2. Device according to claim 1, characterized in that it comprises three elements (8, 9, 10) stacked the one onto the other, means being provided for maintaining together said elements during the displacement of the intermediary element (9) in order to modify on the one hand, the axial shifting of the prosthesis portions (5, 6) and, on the other hand, the relative inclination of said prosthesis portions.

3. Device according to one of claims 1 and 2, characterized in that the elements (8, 9, 10) are connected by magnetic means preventing an accidental pulling-out during the displacement of the intermediary element (9) relatively to the elements (8, 10) fixed to the prosthesis portions (5, 6).

4. A device according to claim 1, characterized in that it comprises a first element (12) fixed to the upper portion (5) of the prosthesis and having a convex bottom face (12a), a second element (14) having on its top a concave face (14a) and on its bottom a convex face (14b), a third element (17) having on its top a concave face (17a) and on its bottom a plane face, a fourth element (20) having on its top and on its bottom a plane face, and a fifth element (24) fixed to the lower portion (6) of the prosthesis and having on its top a plane face.

5. Device according to one of claims 1 and 4, characterized in that the concave and convex faces are parts of cylindrical surfaces.

6. Device according to one of claims 1 to 4, characterized in that the concave and convex faces are spherical surfaces.

7. Device according to one of claims 1 to 6, characterized in that the elements are made of wood, metal, plastics and other materials, and comprise guiding means ensuring their mutual connection.

8. Device according to one of claims 1, 2 and 5, 6, characterized in that the elements have on their contact faces, orthogonal dove tailed guiding means (13, 15, 16, 18, 19, 20, 21, 22, 23).

9. Device according to one of claims 1 to 8, characterized in that it is fixed to the prosthesis elements (5, 6) by a band which is wrapped and impregnated with a plastics material.

10. Method for the alignment, one with respect to the other, of two portions of a prosthesis of an amputated person lower limb carrying into effect the device of one of claims 1 to 9, characterized in that alignment adjusting means are placed between said two portions and in that said alignment adjusting means are immobilized after adjustment so that they become an integral part of the prosthesis, in that adjusting means are moreover placed between said two portions for adjusting the orientation of one of the portions with respect to the other and in that said orientation adjusting means are immobilized after adjustment so that they become an integral part of the prosthesis and in that, further, the outer generating lines of the adjusting means are rectified after their final fixation and in that they are wrapped so that they become an integral part of the prosthesis.

0 102 853

Fig.1

Fig.2

Fig.4

Fig.3

0 102 853

*Fig:5*

*Fig:6*

2

*Fig:7*

*Fig:8*

5

28

8a

30

4a

4

31

6

29

5

4

6

7